# EUROPEAN PATENT APPLICATION

(11) **EP 1 133 986 A1**
(43) Date of publication of application: **19.09.2001**
(21) Application number: 00302207.6
(22) Date of filing: 17.03.2000
(51) Int. Cl.: A61K 31/135

(54) **Use of deramciclane for the treatment of anxiety and depression**

(71) Applicant: ORION CORPORATION, 02200 Espoo (FI)
(72) Inventor: The designation of the inventor has not yet been filed
(74) Representative: Sexton, Jane Helen

(57) **Abstract**

The present invention relates to deramciclane, (1R,2S,4R)-(-)-2-[N,N-(dimethylaminoethoxy)]-2-phenyl-1,7,7-trimethyl-bicyclo[2.2.1]heptane, and its use in the treatment of disorders relating to serotonergic systemin humans, specifically depression and anxiety.

## Description

### BACKGROUND OF THE INVENTION

The present invention is directed to deramciclane, (lR,2S,4R)-(-)-2-[N,N-(dimethylaminoethoxy)]-2-phenyl-1,7,7-trimethyl-bicyclo[2.2.1]heptane, and its use in the treatment of disorders relating to the serotonergic system. Specifically, the present invention is directed to the use of deramciclane in the treatment of depression.

Further, the present invention is directed to the use of deramciclane in the treatment of anxiety, specifically GAD, in an oral daily dose of 30 mg in humans.

The preparation of deramciclane as a free base and as a fumarate salt has been described in Hungarian Patent No. 212,547. Other pharmaceutically acceptable acid addition salts of deramciclane may be formed with inorganic (e.g., hydrochloric acid, sulfuric acid) or organic acids (e.g., acetic acid, tartaric acid). The fumarale salt is preferred.

Deramciclane has shown anxiolytic-like effects in some conventional animal models with various routes of administration, and in receptor binding studies in *vitro* deramciclane has shown to bind with high affinity to serotonin 5HT_{2A}- and 5-HT_{2C}-receptor subtypes, being a potent antagonist of these receptors (Gacsalyi, I. et al., Drug Dv Res (1997) 40:333-348). In punished drinking test in rats (Vogel, J.R. et al., Psychopharmacologia (1971) 21:1-7) deramciclane was active, after single oral administration at doses of 1 mg/kg and 10 mg/kg. In social interaction test in rats (File, S.E. J. Neurosci Methods (1980) 2:219-238) deramciclane enhanced the social interaction time in some extend, the minimum effective dose after single intraperitoneal administration was 0.7 mg/kg. In two compartment test in mice (Crawley, J. and F.K. Goodwin, Pharmacol Biochem Behav. (1980)13, 167-170. & Crawley, J.N., Pharmacol. Biochem. and Behav. (1981) 15, 695-699) deramciclane was active after single subcutaneous administration at dose of 3 mg/kg. In marble-burying test in mice (Broekkamp, C.L. et al., Eur J. Pharmacol. (1986) 126:223-229) the effective doses were 10 mg/kg and 30 mg/kg orally. Nevertheless, deramciclane was totally ineffective in elevated plus maze test in rats (Handley, S.L. and S. Mithani, 1984, Effects of Alpha-Adrenoceptor Agonists and Antagonists in a Maze-Exploration Model of "Fear"-Motivated Behaviour, Naunyn-Schmiedeberg's Archives of Pharmacology. 327, 1-5) after single intraperitoncal doses at range of 0.1 mg/kg - 5 mg/kg. However, deramciclane was able to attenuate the caerulein-induced degrease in exploratory behavior at an intraperitoneal dose of 0.5 mg/kg in the elevated plus maze test.

Also the possible stress protective and antidepressant activity of deramciclane has been evaluated in various conventional animal models. In learned helplessness test in rat (Giral et al. Reversal of helpless behavior in rats by putative 5-HT1A agonists. Biol. psychiatry 23: 237- 242), deramciclane dose dependently attenuated helpess behaviour induced by inescapable electric foot shocks, when given intraperitoneally 1 or 10 mg/kg, repeatedly 8 times, twice a day, before the test. The effect of deramciclane was found to be negligible, even at relatively high oral doses, 48-160 mg/kg, when evaluated for tetrabenazine-induced ptosis in mice according to the method of Howard et al. (Howard, J.L. et al., (1981) Empirical behavioral models of depression with emphasis on tetrabenazine antagonism. In Enna S.J., Malick J.B., Richelson E. (eds.): Antidepressants: Neurochemical, Behavioral, and Clinical Perpectives. New York: Raven Press, p 107). In the forced swimming test in rats (Porsolt R.D. et al., Eur. J. Pharmacol. (1978) 47:379-391) deramciclane was clearly ineffective at oral doses of 25 and 100 mg/kg.

Thus, deramcilane has been effective in some animal models of anxiety after oral doses at range from 1 mg/kg to 30 mg/kg in mice and rats. Further, deramciclane has showed negligible effects in animal models of depression even after high peroral doses in mice and rats, which is in line with the results reporting that 5-HT_{2C} -receptor agonists are effective in animal models of depression (Moreau J-L. et al. European Neuropsychopharmacology 6:169-175, 1996).

In a whole body autoradiography distribution study with tritium labelled deramciclane in rats (Hazai, I, et al. J. Pharm. Pharmacol. 51: 165-174, 1999) at a dose of 3 mg/kg, it was found that after intravenous administration there were high radioactivity (reflecting amount of deramciclane) in several organs including blood and the brain, but after oral administration the amount was substantially lower, especially in the brain.

A comparative pharmacokinetic study of orally administered deramciclane in rats, dogs, rabbits and humans (Klebovich et al Pharm. Pharmacol. Commun., 4: 129-136, 1998), it was shown that the plasma concentration curves obtained after the administration of a single 3mg/kg oral dose of deramciclane to rats (dogs, rabbits) and human show considerable species specific differences. In the peak plasma concentration (Cmax) values there were significant differences: Cmax was 5.4 ng/ml in rat and 217.5 ng/ml in human after the same 3 mg/kg oral dose. Thus a 40-times lower oral dose of deramciclane could be used in man to result in the same maximal plasma concentration as in rat. Furthermore, total amount of deramcilane absorbed into blood, calculated as Area Under Curve values (AUC 0 - ∞) from plasma concentrations as a function of time, showed more considerable species difference. The mean AUC 0 - ∞ values after single oral administration of deramciclane were 11.9 ng h/ml and 3737.8 ng h /ml in rat and human, respectively. Thus over 300 times lower oral doses should result in equal exposure in humans than in rats. Basing only the Cmax difference between rat and man, it can be predicted that considerably lower doss should be centrally acitive in humans than in rat. The minimum oral effective anti-anxiety dose in rats was 1 mg/kg (1-30mg/kg the full range; see above), i.e. in a 70 kg-man this would mean 70 mg dose. To reach the same pharmacologically active plasma concentration in human as was shown to be efficacious in rat, one should divide the rat dose by 40. This would result in 70 mg/40= 1.75 mg (i.e. 0.025 mg/kg) as an effective dose in man.

The binding of deramciclane to serotonin 5-HT_{2A}-receptors in frontal cortex of healthy male volunteers after a single oral dose of 20, 50 and 150 mg of deramciclane is discussed in Kanerva, H., et al., Psychopharmacology (1999) 145:76-81. The determination of the brain 5HT_{2A} -receptor occupancy of deramciclane in humans has shown that 90% and 50% receptor occupancies were reached at deramciclane plasma concentration of about 70 ng/ml and 21 ng/ml, respectively. The pharmacokinetics of single dose of deramciclane and during oral dosing of 10 mg, 30mg and 60mg twice a day for seven days are discussed in Kanerva, H., Pharmacokinetic studies on deramciclane. Kuopio University Publications A. Pharmaceutical Sciences 39.1999. After a single oral administration of 20 mg and 30 mg doses of deramciclane, the Cmax-values were 24±9.4 ng/ml and 27±6.1 ng/ml, respectively. During repeated administration of deramciclane for one week the Cmin and Cmax for 60 mg and 20 mg daily doses were shown to range between 48-91 ng/ml and 16-33 ng/ml, respectively.

As can be seen from the above experimental animal and human data, it was impossible to foresee the exact oral doses of deramciclane effective in treating anxiety in humans. Furthermore, it was totally unexpected that deramciclane was effective in treating depressive symptoms.

Anxiety is a normal emotional feeling related to different situations of threat or fear. External threat is experienced as a fear whereas obscure and unidentified feeling of threat may be experienced as anxiety. When anxiety persists it can develop into a pathological disorder. Anxiety disorders are divided more specifically in diagnostic disorders e.g., panic disorder, phobias, and generalized anxiety disorder (GAD). GAD is a chronic illness associated with excessive anxiety and worry lasting for at least six months. In addition, the anxiety and worry are associated with restlessness, fatigue, difficulties in concentrating or mind going blank, irritability, muscle tension, and sleeping disturbances. The symtomps may be triggered by different events of life, and the control of anxiety is very difficult to the patient. GAD is currently treated with benzodiazepines and buspirone, which are not optimal treatments regarding the adverse drug reaction and efficacy profiles.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE DRAWINGS

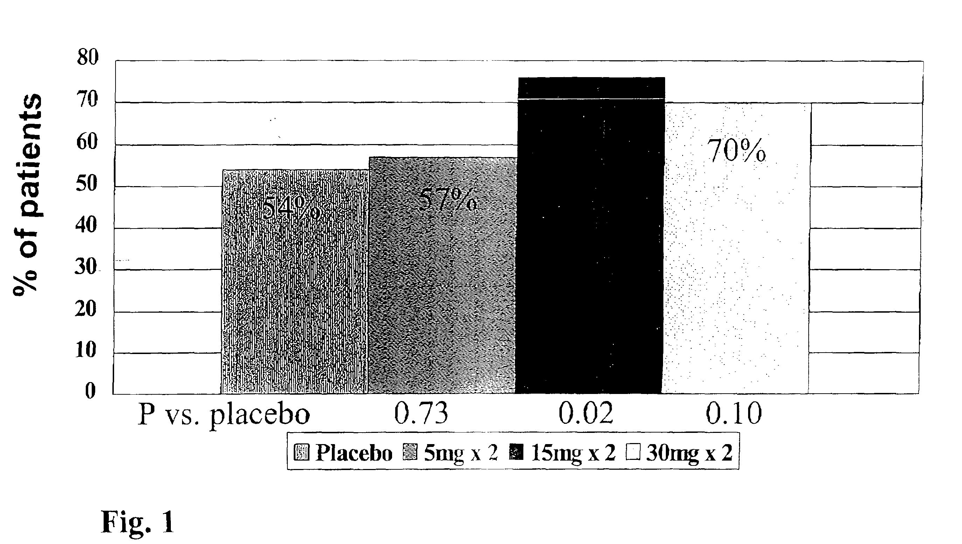

Figure 1 shows the number of responders (at least 50 % reduction in HAM-A scale).

### DETAILED DESCRIPTION OF THE INVENTION

In the present invention it has been discovered that when treating disorders relating to anxiety in humans with deramciclane the most effective clinical response was obtained with an oral daily dose of 30 mg.

When deramciclane was used in the treatment of anxiety, specifically GAD, the oral daily dose of 30 mg was found to express the best response on Hamilton Anxiety Scale (HAM-A). Further, in the pairwise comparison of the responder criterion (at least 50 % reduction in HAM-A scale) between the deramciclane dose and placebo, the oral daily dose of 30 mg was statistically better than placebo.

In addition, in the present invention it has been discovered that deramciclane is effective in treating depressive disorders in humans. Specifically, the same oral daily deramciclane dose of 30 mg which was effectivc in the tretment of anxiety was found to be effective on the depressive symtoms in the Montgomery Åsberg Depression Rating Scale (MADRS).

The daily dose of 30 mg could be divided into two 15 mg dosages or it could be administered on once daily basis.

### EXAMPLE

The efficacy of deramciclane in the treatment of anxiety, specifically GAD, was studied in a randomised placebo-controlled double-blind study. In addition, the dose-dependency of the effects of deramciclane was evaluated. Total of 208 patients were included in the study. The subjects were randomly assigned to four parallel groups to receive one tablet twice daily (b.i.d) of a placebo, 5mg (=10mg/day), 15mg (=30mg/day), or 30mg (=60mg/day) deramciclane. The study started with a one-week placebo run-in period, followed by an eight-week placebo-controlled active treatment and a two-week placebo washout period.

The efficacy of deramciclane on anxiety symptoms was studied by the efficacy variable Hamilton Anxiety Scale (HAM-A), analyzing the change in the score and using responder criterion (at least 50 % reduction in HAM-A total score).

The efficacy of deramciclane on depressive symptoms was studied using Montgomery Åsberg Depression Rating Scale (MADRS).

### RESULTS

### Anxiety

The HAM-A score decreased 14.5 points from baseline in groups receiving either 15 mg b.i.d or 30 mg b.i.d. dose. However, only the 15 mg b.i.d group differed statistically significantly from placebo (p=0.006). No difference was found between the 5 mg b.i.d and placebo. Accordingly, increasing the dose over 15mg b.i.d did not increase the efficacy any more.

Number of responders (at least 50 % reduction in HAM-A scale) is presented in Figure 1. The responder criterion was reached by 54 % (n=27), 57% (n=31), 76% (n=39) and 70% (n=37) of patients on placebo, 5 mg, 15 mg and 30 mg b.i.d. dosing, respectively. In comparison between the deramciclane dose levels and placebo, only 15 mg b.i.d. was statistically better than placebo (p=0.020).

### Depression

MADRS scores decreased similarily and statistically significantly in both 15mg b.i.d and 30 mg b.i.d groups (7.7 and 8.0 points, p= 0.028 and 0.016, respectively). 5 mg b.i.d dose was ineffective. Thus, both the oral daily doses of 30 mg and 60 mg of deramciclane were found to be effective on the depressive symptoms.

Although the invention has been illustrated by the preceding example, it is not to be construed as being limited to the materials employed therein; rather, the invention is directed to the generic area as herein disclosed. Various modifications and embodiments thereof can be made without departing from the spirit or scope thereof.

## Claims

1. Use of deramciclane in the manufacture of a medicament for the treatment of disorders relating to the serotonergie system in humans in an oral daily dose of 30 mg.

2. The use according to claim 1, wherein the disorder is anxiety.

3. The use according to claim 2, wherein the disorder is generalized anxiety disorder.

4. Use of deramciclane in the manufacture of a medicament for the treatment of disorders relating to depression in humans.

5. The use according to claim 4, wherein an oral daily dose of 30 mg is used.
